# EUROPEAN PATENT SPECIFICATION

(11) **EP 1 417 955 B1**
(45) Date of publication and mention of the grant of the patent: **05.09.2007**
(21) Application number: 02760624.3
(22) Date of filing: 13.08.2002
(51) Int. Cl.: A61K 8/18, A61K 31/047, A61K 31/23, A61P 17/16

(54) **HUMECTANT AND COSMETICS AND EXTERNAL PREPARATIONS CONTAINING THE SAME**
BEFEUCHTUNGSMITTEL UND DIESES ENTHALTENDE KOSMETIKA UND TOPISCHE PRÄPARATE
HUMECTANT ET COSMETIQUES ET PREPARATIONS EXTERNES CONTENANT CET HUMECTANT

(30) Priority: 13.08.2001 JP 2001245282
(43) Date of publication of application: 12.05.2004
(73) Proprietor: The Nisshin OilliO Group, Ltd., Tokyo 104-8285 (JP)
(72) Inventor: FUJINO, Jin, Yokohama-shi, Kanagawa 235-0023 (JP); OOYAMA, Keiichi, Yokohama-shi, Kanagawa 235-0023 (JP)
(74) Representative: Vuillermoz, Bruno
(86) International application number: PCT/JP2002/008255
(87) International publication number: WO 2003/015741

(56) References cited:
- WO-A-00/02529
- JP-A- 9 100 225
- JP-B2- 60 021 568
- JP-B2- 61 007 168
- US-A- 5 658 575
- US-A- 5 919 398
- US-A- 5 925 364
- US-A- 5 951 991

## Description

### TECHNICAL FIELD

The present invention relates to a humectant comprising a specific component, and a cosmetic and an external agent comprising the same and, more particularly, to a humectant which is excellent in stability at high temperature, and a cosmetic and an external agent comprising the same.

### BACKGROUND ART

It has been well known that moisture is deeply involved in formulation components of various products such as foods, cosmetics, agricultural chemicals, feeds, and medicaments, and a moisture retaining function in these products is one of important quality functions. Examples of a known humectant used in cosmetic compositions or external agents include polyhydric alcohols such as glycerin, 1,3-butylene glycol, and sorbitol. In addition, main components of NMF (natural moisturizing factor) such as pyrrolidone carboxylate and lactate, and sodium hyaluronate obtained by microbial production are also used.

The humectant plays such an important role that it serves as a moisture retaining agent of the products and also contributes to retention of stability of the system. Because of excellent moisture retaining properties and excellent moisture retaining properties, polyhydric alcohols, which are trihydric or above, such as glycerin and sorbitol are used as a humectant having highest general-purpose properties in view of safety, stability and cost.

A necessary condition for the humectant is that it can retain moisture for a long time without being influenced by environmental conditions (for example, temperature, humidity, wind, etc.), especially ambient humidity. In any humectant, however, a moisture absorption amount and a moisture release amount are influenced by ambient humidity due to a relation of a vapor pressure. In case of glycerin, an equilibrium moisture content is 60% under the conditions of a temperature of 25°C and a relative humidity of 75%, and the equilibrium moisture content is 15% at a relative humidity of 33%. Also in case of sorbitol, the equilibrium moisture content is 50% at a relative humidity of 75%, and the equilibrium moisture content is 5% at a relative humidity of 33%. As described above, the moisture content varies depending on the ambient relative humidity.

Namely, the humectant of the prior art has such a problem that it releases moisture at low ambient relative humidity, resulting in low moisture content. Therefore, it is necessary for the humectant that it slowly transpires moisture under the environment of low humidity. Taking a serious view of transpiration of moisture under the environment of low humidity, lecithin is widely used at present. However, it has drawbacks, for example, easy-to-deposit properties at an isoelectric point under low pH, poor salt resistance, decrease in viscosity caused when used in combination with cetanol, easy-to-hydrolyze properties, and poor heat resistance.

Cosmetics and external agents have recently been circulated as products, and thus a new problem such as stability at high temperature has arisen. A conventional guarantee system in the circulation of cosmetics and external agents required stability at 40°C. Therefore, a storage test of cosmetics and external agents is conducted at 40°C and also an acceleration test is conducted at 40°C. However, as a result of warming due to ozone layer depletion, and urban warming, a mean temperature increases. Particularly, in the suburbs of an urban area, the temperature drastically increases at daytime in the summer season, and the temperature often reaches 35°C or higher. Therefore, the temperature often reaches 40°C or higher in a room with no air-conditioning system, a bag while being carried, and a car. Under the conditions at high temperature, the product becomes drastically soft and jumps out vigorously when a container is opened, or leakes out of the container. Furthermore, an oil layer is often separated.

Therefore, it is required to develop a humectant having a moisture retaining function which is less likely to be influenced by environmental conditions, and a cosmetic and an external agent which have excellent viscosity retaining properties and excellent storage stability even at high temperature of 40°C or higher.

An emulsion-based preparation has conventionally been designed while covering up defects thereof so that the resulting preparation satisfies required physical properties and has desired tactile sensation. However, it was very difficult to formulate a preparation which has excellent moisture retaining properties, and also has excellent stability at high temperature, which enables retention of high viscosity at high temperature of 40°C or higher.

Documents US-A-5 951 991, US-A-5 658 575, US-A-5 925 364, WO 00/02529 A, US-A-5 919 398, JP 60 021 568 B2, JP 61 007 168 B2 and JP-A-9 100 225 report cosmetic compositions deriving from glycerin (or glycerol), which properties remain to be improved.

### DISCLOSURE OF THE INVENTION

An object of the present invention is to solve the above problems and to provide a humectant which is excellent in moisture retaining properties and stability at high temperature, and a cosmetic and external agent comprising the same.

As used herein, the term "moisture retaining properties" refer to properties capable of retaining nearly fixed moisture regardless of high temperature or low temperature, and high humidity or low humidity, while the term "stability at high temperature" refer to properties capable of retaining high viscosity at high temperature of 40°C or higher and existing in a stable state.

Under these circumstances, the present inventors have bukle down to the development from a viewpoint different from that of the prior art so as to improve moisture retaining properties and stability at high temperature in an oil-in-water type emulsion-based preparation. Namely, they have intensively studied to develop a humectant which exhibits moisture retaining properties by adding to an emulsion-based substance in a small amount, and is also suited for use in various emulsion-based substances and has excellent stability at high temperature. As a result, they have found a humectant which is excellent in moisture retaining properties and is also excellent in stability at high temperature, especially 40°C or higher, and thus the present invention has been completed. The present inventors have also found that a cosmetic composition and an external agent, which contain the humectant of the present invention, are excellent in moisture retaining properties and stability at high temperature, like the humectant.

The present invention provides a humectant comprising a component A: an ester compound comprising a condensate of glycerin having polymerization degree of 5 or more, a straight chain saturated fatty acid having 16-28 carbons, and an aliphatic saturated dicarboxylic acid having 16-28 carbons, more than half of the hydroxyl groups of the condensate of glycerin remaining as hydroxyl groups in the ester compound; a component B: a dihydroxy water soluble alcohol; and a component C: a polyhydroxy water soluble alcohol which is trihydroxy or above.

Also, the present invention provides a cosmetic comprising the humectant.

Furthermore, the present invention provides an external agent comprising the humectant.

### BEST MODE FOR CARRYING OUT THE INVENTION

The humectant of the present invention is a humectant comprising the following components A, B and C.

That is, the humectant is a humectant comprising
a component A: an ester compound comprising a condensate of glycerin having polymerization degree of 5 or more, a straight chain saturated fatty acid having 16-28 carbons, and an aliphatic saturated dicarboxylic acid having 16-28 carbons, more than half of the hydroxyl groups of the condensate of glycerin remaining as hydroxyl groups in the ester compound;
a component B: a dihydroxy water soluble alcohol; and
a component C: a polyhydroxy water soluble alcohol which is trihydroxy or above.

The humectant of the present invention comprising the above components has high moisture retaining properties and also has excellent stability at high temperature.

Also the present invention relates to a humectant wherein the ester compound as the component A is an ester compound comprising a condensate of glycerin, a straight chain saturated fatty acid having 16-28 carbons, and an aliphatic saturated dicarboxylic acid having 16-28 carbons, and is obtained by an esterification reaction of them in a charge amount which enables more than half of the hydroxyl groups of the condensate of glycerin to remain as hydroxyl groups in the ester compound in case of ester bonding of the condensate of glycerin with the straight chain saturated fatty acid and the aliphatic saturated dicarboxylic acid.

First, the ester compound used as the component A in the present invention will be described.

To prepare the ester compound as the component A, a condensate of glycerin having polymerization degree of 5 or more is used as an essential component. Namely, a condensate of glycerin, or a mixture of glycerin condensate is used as an essential component.

As used herein, the term "condensate of glycerin" means polyglycerin having a mean polymerization degree of 5 or more. Since the polyglycerin having higher mean polymerization degree has higher hydrophilicity and higher moisture retaining ability, polyglycerin having a mean polymerization degree of 5 or more is more preferable and polyglycerin having a mean polymerization degree of about 10 is most preferable. Specific examples thereof include pentaglycerin, hexaglycerin, and decaglycerin, and these polyglycerins can be used alone or in combination.

It is indispensable that the straight chain saturated fatty acid (straight chain saturated aliphatic monocarboxylic acid) is a straight chain saturated fatty acid having 16-28 carbons. The straight chain saturated fatty acid is indispensably a straight chain saturated fatty acid having 16-28 carbons, but is preferably a straight chain saturated fatty acid having 18-24 carbons, and most preferably a straight chain saturated fatty acid having 20 or 22 carbons. The straight chain saturated fatty acid having carbons of less than 16 is not preferable because the resulting humectant containing an ester compound formulated therein bas poor storage stability and is likely to cause separation with a lapse of time. To the contrary, the straight chain saturated fatty acid having carbons of more than 28 is not preferable because it becomes difficult to perform deodorization and decolorization. The branched saturated fatty acid or unsaturated fatty acid is not preferable because the resulting humectant containing an ester compound formulated therein has poor storage stability and is likely to cause separation with a lapse of time.

Examples of the straight chain saturated fatty acid, which can be preferably used in the present invention, include palmitic acid, stearic acid, 10-hydroxystearic acid, 10-ketostearic acid, 1 2-hydroxystearic acid, arachidic acid, behenic acid, and montanoic acid, and these straight chain saturated fatty acids can be used alone or in combination. The humectant obtained by formulating an ester compound containing fatty acid having a lot of carbons has improved storage stability. Taking account of availability of raw materials and difficulty of synthesis of an ester compound due to an increase in melting point, behenic acid is most preferable.

It is indispensable that the saturated aliphatic dicarboxylic acid is an aliphatic saturated dicarboxylic acid having 16-28 carbons. The saturated aliphatic dicarboxylic acid is indispensably an aliphatic saturated dicarboxylic acid having 16-28 carbons, but is preferably an aliphatic saturated dicarboxylic acid having 18-24 carbons and an aliphatic saturated dicarboxylic acid having 20 or 22 carbons is most preferable. The humectant containing an ester compound (component A), which is obtained from an unsaturated aliphatic dicarboxylic acid or a saturated aliphatic dicarboxylic acid having carbons of less than 16, formulated therein has poor storage stability and is likely to cause separation with a lapse of time. The saturated aliphatic dicarboxylic acid having carbons of more than 28 is not easily available as an industrial material,

Examples of the saturated aliphatic dicarboxylic acid, which can be preferably used in present invention, include eicosanedicarboxylic acid, docosacosadicarboxylic acid, tetracosadicarboxylic acid, hexacosadicarboxylic acid, and octacosadicarboxylic acid, and these saturated aliphatic dicarboxylic acids can be used alone or in combination. The humectant obtained by formulating an ester compound containing dicarboxylic acid having a lot of carbons has improved storage stability. Taking account of availability of raw materials and difficulty of synthesis of an ester compound due to an increase in melting point, eicosadicarboxylic acid is most preferable among these saturated aliphatic dicarboxylic acids.

The ester compound as the component A can be obtained by the following method using the above raw materials in combination.

Namely, a desired component A can be obtained by simultaneously oligoesterifying a condensate of glycerin, a straight chain saturated fatty acid having 16-28 carbons and an aliphatic saturated dicarboxylic acid having 16-28 carbons. Also it can be obtained by esterifying a condensate of glycerin with a straight chain saturated fatty acid having 16-28 carbons and oligoesterifying or transesterifying the resulting product with an aliphatic saturated dicarboxylic acid having 16-28 carbons. Furthermore, it can be obtained by oligoesterifying a condensate of glycerin with an aliphatic saturated dicarboxylic acid having 16-28 carbons and esterifying the resulting product with a straight chain saturated fatty acid.

The esterification reaction is preferably conducted in an organic solvent and/or a gas, which are inert to the above reaction, under the presence of absence of an acid catalyst, an alkali catalyst, or a metal catalyst at 100 to 240°C for several hours to 20 hours while removing water produced as by-product. The transesterification reaction is preferably conducted at 20 to 140°C for several tens of minutes to several tens of hours, using a catalyst such as metal alcoholate or lipase. The reaction progress can be judged by measuring the acid value in the system or the composition of the isolated acid component, whereby, a reaction completion point may be decided. When the reaction is conducted until the acid value becomes 5 or less, preferably 1 or less, and a decrease in acid value is terminated, it is made possible to obtain an ester compound wherein almost all of the straight chain saturated fatty acid and the saturated aliphatic dicarboxylic acid charged as raw materials are esterified. A purified ester compound can be obtained by subjecting the resulting ester compound to treatments such as removal of the solvent, decolorization, and deodorization.

The ester compound thus obtained is a mixture wherein a condensate of glycerin, a straight chain saturated fatty acid and an aliphatic saturated dicarboxylic acid are oligoesterified in the form of a straight chain and/or a network, and has a melting point of about 50 to 80°C.

It is indispensable that the ester compound used in the present invention is an ester compound in which more than half of the hydroxyl groups of the condensate of glycerin, as raw materials, remain. When the remaining hydroxyl groups of the ester compound are less than half of the hydroxyl groups, the ester compound (component A) has poor compatibility with the polyhydroxy alcohol (components B and C) and an insoluble matter is likely to deposit with a lapse of time during storage of the humectant, unfavorably.

The number of the hydroxyl groups of the glycerin condensate is determined based on a mean polymerization degree, and the mean polymerization degree is determined by calculating based on the hydroxyl value. In case of a glycerin condensate having a mean polymerization degree of n (n is an integer), the number of half of the hydroxyl groups in 1 mol is (n + 2)/2. In the esterification reaction, 1 mol of the straight chain saturated fatty acid is ester-bonded with a hydroxyl group of the condensate of glycerin, while 1 mol of the aliphatic saturated dicarboxylic acid is ester-bonded with two hydroxyl groups of the condensate of glycerin.

Therefore, the ester compound in which more than half of the hydroxyl groups of the condensate of glycerin, as raw materials, remain can be obtained by changing the proportion of the charged raw materials based on the proportion of the charged raw materials of the ester compound in which half of the hydroxyl groups of the condensate of glycerin remain. Specifically, the proportion, which give an ester compound in which just half of the hydroxyl groups of the condensate of glycerin remain when a condensate of glycerin and all of straight chain saturated fatty acid and aliphatic saturated dicarboxylic acid are esterified, that is, a molar amount of raw materials is set, first. When a predetermined molar amount of a condensate of glycerin and a predetermined molar amount of a straight chain saturated fatty acid and an aliphatic saturated dicarboxylic acid are charged as raw materials and they are esterified until the acid value becomes 5 or less, preferably 1 or less, and a decrease in acid value is terminated, it is made possible to obtain an ester compound in which half of the hydroxyl groups of the condensate of glycerin remain. Consequently, when a predetermined molar amount of a condensate of glycerin and a molar amount smaller than a predetermined molar amount of a straight chain saturated fatty acid or an aliphatic saturated dicarboxylic acid are charged as raw materials and then esterified until the above conditions are satisfied, it is made possible to obtain an ester compound in which more than half of the hydroxyl groups of the condensate of glycerin remain.

In case of an ester compound comprising decaglycerin (hydroxyl value: 882.2), behenic acid and eicosadicarboxylic acid, a theoretical charge amount which gives an ester compound in which half of the hydroxyl groups of the condensate of glycerin remain is as follows: for example, decaglycerin: 1 mol, behenic acid: 5 mol, and eicosadicarboxylic acid: 0.5 mol. In this case, when decaglycerin is ester-bonded with all of behenic acid and eicosadicarboxylic acid, half of the hydroxyl groups of decaglycerin remains. In this theoretical charge amount, when the amount of behenic acid is decreased and decaglycerin is ester-bonded with all of behenic acid and eicosadicarboxylic acid, more than half of the hydroxyl groups of decaglycerin remain.

Examples of the ester compound as the component A, which is preferably used in the present invention, include decaglycerin fatty acid ester eicosane diacid condensate (manufactured by The Nisshin Oil Mills, Ltd. under the trade name of Nomcort HK-P).

The dihydroxy water soluble alcohol used as the component B in the present invention will now be described. The dihydroxy water soluble alcohol used as the component B is not specifically limited as far as it is a water soluble alcohol having two hydroxyl groups in a molecule. Typical examples thereof include 1,2-pentanediol, hexanediol, 1,3-butylene glycol, and propylene glycol, and these dihydroxy water soluble alcohols can be used alone or in combination. Among these dihydroxy water soluble alcohols, 1,3-butylene glycol is most preferable because of high compatibility with the ester compound. It is simple to use, as 1,3-butylene glycol, a commercially available product (manufactured by DAICEL CHEMICAL INDUSTRIES, LTD.).

The water soluble polyhydroxy alcohol which is trihydroxy or above, that is used as the component C in the present invention, will now be described, The water soluble polyhydroxy alcohol which is trihydroxy or above, that is used as the component C, is not specifically limited as far as it is a water soluble polyhydroxy alcohol having three or more hydroxyl groups in a molecule. Typical examples thereof include glycerin, diglycerin, triglycerin, polyglycerin, neopentyl glycol, sorbitol, erythritol, pentaerythritol, glucose, galactose, fructose, sucrose, maltose, xylose, xylobiose, and a reduced compound of oligosaccharide, and these water soluble polyhydroxy alcohols can be used alone or in combination. Among these water soluble polyhydroxy alcohols which are trihydroxy or above, glycerin is preferable because the humectant is easily gelatinized.

In the humectant of the present invention, the content of the ester compound as the component A is from 0.1 to 50% by mass, preferably from 0.1 to 40% by mass, and particularly preferably from 0.5 to 30% by mass, based on 100% by mass of the humectant. When the content of the ester compound is less than 0.1 % by mass, the resulting humectant may have poor moisture retaining properties. On the other hand, when the content exceeds 50% by mass, the ester compound is not uniformly dissolved in the humectant and an insoluble matter is likely to deposit, unfavorably.

The total content of the components B and C is from 5 to 99.9% by mass, and preferably from 10 to 95% by mass, based on 100% by mass of the humectant. The total content of the components B and C of less than 5% by mass is not preferable because the resulting humectant may have poor moisture retaining properties. Furthermore, a mass ratio of the dihydroxy water soluble alcohol as the component B to the water soluble polyhydroxy alcohol which is trihydroxy or above as the component C is preferably from 1:0.1 to 1:20, and more preferably from 1:0.1 to 1:10. When the proportion of the component C is less than 0.1 based on the proportion 1 of the component B, satisfactory moisture retaining properties are not achieved. The proportion of the component C of more than 20 based on the proportion 1 of the component B is not preferable because the ester compound (component A) can not be stably dissolved in the water soluble polyhydroxy alcohol (components B and C) and precipitation is likely to occur at room temperature to low temperature, for example, 20°C to 5°C.

The method of preparing the humectant of the present invention is not specifically limited and the humectant can be prepared, for example, by heating an ester compound as the component A, a dihydroxy water soluble alcohol as the component B, and a water soluble polyhydroxy alcohol which is trihydroxy or above as the component C to a temperature of 60°C to 90°C, preferably 70°C to 80°C, and uniformly dissolving the respective components while stirring for about 10 minutes to one hour using a bar, a propeller, a homomixer, or a dispersing mill. In case of stirring the component A, the component B and the component C at a temperature lower than 60°C, they can not be uniformly mixed, sometimes. In case of stirring at a temperature higher than 90°C, the respective components are decomposed by heat, unfavorably.

The humectant containing the component A, the component B and the component C of the present invention may not contain water or may contain water. The amount of water is preferably from 0.1 to 80% by mass, more preferably from 1 to 50% by mass, and most preferably from 1 to 20% by mass, based on 100% by mass of the total mass after adding water. Even if the amount of water exceeds 80% by mass based on 100% by mass of the total mass after the addition of water, further moisture retaining effect can not be expected.

In case the water soluble polyhydroxy alcohol which is trihydroxy or above (component C) is a solid saccharide such as sucrose or glucose, it becomes difficult to uniformly mix with the ester compound. Therefore, water is preferably added in the amount of 10 to 80% by mass, more preferably 30 to 80% by mass, and most preferably 60 to 80% by mass, based on 100% by mass of the total mass after the addition of water.

The humectant comprising the component A, the component B and the component C can further contain an oily substance. To the humectant, water and the oily substance can also be added. By adding the oily substance, moisture retaining properties and stability at high temperature can be further improved. The amount of the oily substance is preferably from 1 to 80% by mass, and more preferably from 10 to 70% by mass, based on 100% by mass of the humectant after the addition of the oily substance. It is not preferable to add the oily substance in the amount of more than 80% by mass based on 100% by mass of the humectant after the addition of the oily substance, because separation of oil is likely to occur.

Examples of the oily substance include, but are not limited to, hydrocarbons, esters, oils and fats, waxes, higher fatty acid, higher alcohol, silicone substances, sterols, and resins. Specific examples thereof include liquid paraffin, isoparaffin, vaseline, squalane, isopropyl myristate, octyldodecyl myristate, cetyl isooctylate (cetyl 2-ethylhexanoate), glyceryl triisooctylate (glyceryl tri-2-ethylhexanoate), glyceryl tricaprylate, diisooctylic acid neopentyl glycol ester (di-2-ethylhexanoic acid neopentyl glycol ester), diisostearyl malate, isononyl isononanoate (3,5,5-trimethylhexanoic acid 3,5,5-trimethylhexyl alcohol ester), cholesteryl 12-hydroxystearate, mono- or hexaisostearic acid dipentaerythritol ester using isostearic acid manufactured by Emery Co., isooctyl o-, m- or p-methoxy cinnamate, eucalyptus oil, soybean oil, cotton seed oil, sesame oil, rice germ oil, rice bran oil, safflower oil, sunflower oil, palm oil, olive oil, jojoba oil, macadamia nuts oil, avocado oil, castor oil, evening primrose oil, turtle oil, mink oil, orange raffia oil, lanoline, myristic acid, palmitic acid, stearic acid, oleic acid, 12-hydroxystearic acid, behenic acid, stearyl alcohol, oleyl alcohol, cetanol, lanoline alcohol, paraffin wax, microcrystalline wax, ceresin wax, beeswax, carnauba wax, candelilla wax, shellac wax, soybean hydrogenated oil, rapeseed hydrogenated oil, glyceryl tristearate, rosin, cholesterol, phytosterol, dimethylpolysiloxane, dimethylpolysiloxane, methylphenylpolysiloxane, and essential oil derived from animals and vegetables. These oily substances can be used alone or in combination. Among these oily substances, higher alcohols such as cetanol are preferable because they exhibit synergism to the ester compound as the component A and viscous properties as the humectant remarkably increase.

In case the humectant of the present invention contains water or an oily substance, the humectant can be obtained by heating the component A, the component B and the component C, and adding water or the oily substance upon stirring the components.

Forms of the humectant of the present invention will now be described.

The humectant of the present invention can take various forms such as liquid form, viscous form such as viscous liquid, and gel form such as semi-solid having no fluidity.

The humectant of the present invention can be used in various forms. When a gel humectant is used as a base, it is made possible to prepare a gel cosmetic composition or external agent. With respect to a humectant containing no oily substance formulated therein, a viscous humectant or a gel humectant is preferable because it is excellent in moisture retaining properties and stability at high temperature. Among these humectants, a gel humectant is most preferable because it is excellent in moisture retaining properties and stability at high temperature.

A non-aqueous humectant containing the component A, the component B and the component C of the present invention is a gel humectant. In case water is formulated in the humectant containing the component A, the component B and the component C of the present invention in the amount of 0.1 to about 50% by mass based on 100% by mass of the total mass after the addition of water, the humectant retains a gel form. The gel humectant is converted into a liquid or viscous humectant by formulating about 50% by mass of water. It is described that the gel humectant is converted into a liquid or viscous humectant by formulating about 50% by mass of water, however, the amount of water required to conversion varies depending on a formulating ratio of the component A, the component B and the component C.

When the oily substance is formulated in the humectant containing the component A, the component B and the component C of the present invention in the amount, which is not more than 10 times (by mass) as much as the humectant, the non-aqueous humectant is a gel humectant. Also when the oily substance is formulated in the humectant containing the component A, the component B and the component C of the present invention in the amount, which is not more than 10 times (by mass) as much as the humectant, and water is further formulated in the amount of 0.1 to about 40% by mass based on 100% by mass of the total mass after the addition of water, the humectant retains a gel form. The gel humectant is converted into a liquid or viscous humectant by formulating about 40% by mass of water. It is described that the gel humectant is converted into a liquid or viscous humectant by formulating about 40% by mass of water, however, the amount of water required to conversion varies depending on a formulating ratio of the component A, the component B and the component C.

The amount of the oily substance to be formulated in the humectant containing the component A, the component B and the component C of the present invention is preferably I to 20 times (by mass) as much as the humectant. When the amount of the oily substance is one time (by mass) as much as the humectant, it is difficult to exert a sensory improving effect by the addition of the oily substance. On the other hand, when the amount is more than 20 times (by mass), separation of oil is likely to occur, unfavorably.

Since the liquid or viscous humectant obtained by formulating the oily substance and water in the humectant containing the component A, the component B and the component C of the present invention contains the oily substance, it becomes an oil-in-water type emulsion state or a solubilized state. As used herein, the oil-in-water type emulsion humectant refers to an emulsion humectant wherein emulsion particles having a mean particle size of 50 nm or more exist in water. The soluble humectant refers to a humectant with transparent liquid appearance obtained by solubilizing a water insoluble substance, thereby to thermodynamically stabilize the water insoluble substance, or a transparent or translucent humectant wherein fine oil particles having a mean particle size of 50 nm or less such as microemulsion are dispersed in water.

The oil-in-water type emulsion humectant will now be described.

The oil-in-water type emulsion humectant can be obtained by adding water to the previously described gel humectant containing the component A, the component B, the component C and the oily substance under stirring. Also the oil-in-water type emulsion humectant can be obtained by further adding water to a gel humectant containing the component A, the component B, the component C, water and the oily substance under stirring.

To obtain the oil-in-water type emulsion humectant, water is preferably added under stirring so that the moisture content becomes 50 to 99% by mass, and preferably 60 to 90% by mass, based on 100% by mass of the oil-in-water type emulsion humectant. The moisture content of more than 99% by mass is not preferable because the emulsion state becomes poor. Before the addition of water under stirring, water soluble and water dispersible components such as known surfactants and humectants may be formulated in the humectant of the present invention. An effect of further improving stability with a lapse of time and moisture retaining ability of the humectant can be exerted by formulating water soluble and water dispersible components such as surfactants and humectants.

The resulting oil-in-water type emulsion humectant can be used as a cosmetic or an external agent as it is, and can also be used as a base for aqueous products and cosmetics.

The solublized humectant will now be described.

When water is added to the previously described gel humectant containing the component A, the component B, the component C and the oily substance under stirring, the viscosity gradually decreases, and thus a solubilized humectant can be obtained. A solubilized gel humectant can also be obtained by adding water to the humectant containing the component A, the component B, the component C, water and the oily substance under stirring.

To obtain a solubilized humectant, water is preferably added under stirring so that the moisture content becomes 50% by mass or more, and preferably from 60 to 90% by mass, based on 100% by mass of the solubilized humectant.

Before the addition of water under stirring, water soluble and water dispersible components such as known surfactants and humectants may be formulated in the humectant of the present invention. An effect of further improving stability with a lapse of time and moisture retaining ability of the humectant can be exerted by formulating water soluble and water dispersible components such as surfactants and humectants.

The resulting solubilized humectant can be used as a cosmetic or an external agent as it is, and can also be used as a base for aqueous products and cosmetics.

The humectant of the present invention is excellent in moisture retaining properties and stability at high temperature even if it takes any forms such as liquid form, viscous form, gel form, oil-in-water type emulsion form and a solubilized form. By making use of its excellent stability at high temperature and various forms, it is made possible to apply to various products in various fields, for example, moisture feeders to flowers in the field of horticulture, improvers for desert soil and dry soil in the field of civil engineering, and humectants in the filed of medical care and cosmetics. According to the purposes, commonly used known components can be formulated in final products.

When the cosmetic contains the previously described humectant containing the component A, the component B and the component C, or the humectant further containing water formulated therein, the content of the humectant is preferably from 0.1 to 90% by mass, and more preferably from 0.1 to 50% by mass, based on 100% by mass of the cosmetic as the final product.

When the cosmetic contains the gel humectant containing the oily substance formulated therein, the content of the gel humectant is preferably from 0.1 to 50% by mass, and more preferably from 0.1 to 40% by mass, based on 100% by mass of the cosmetic as the final product.

When the cosmetic contains the oil-in-water type emulsion humectant or solubilized humectant, the content of the humectant is preferably from 0.1 to 100% by mass, and more preferably from 1 to 100% by mass, based on 100% by mass of the cosmetic as the final product.

Examples of the cosmetic include cosmetic compositions such as cream, emulsion, beauty wash, cosmetic essence, and cleansing gel; external agents such as ointment and gel; skin care cosmetic compositions such as moisture gel and pack; and makeup cosmetics whose moisture retaining effect is expected, such as emulsion foundation, emulsion eye shadow, and nail treatment.

Furthermore, the present invention provides an external agent containing the humectant.

When the external agent contains the previously described humectant containing the component A, the component B and the component C, or the humectant further containing water formulated therein, the content of the humectant is preferably from 0.1 to 90% by mass, and more preferably from 0.1 to 50% by mass, based on 100% by mass of the external agent as the final product.

When the external agent further contains the gel humectant containing the oily substance formulated therein, the content of the gel humectant is preferably from 0.1 to 50% by mass, and more preferably from 0.1 to 40% by mass, based on 100% by mass of the external agent as the final product.

When the external agent contains the oil-in-water type emulsion humectant or the solubilized humectant, the content of the oil-in-water type emulsion humectant or the solubilized humectant is preferably from 0.1 to 100% by mass, and more preferably from 1 to 100% by mass, based on 100% by mass of the external agent as the final product.

Examples of the external agent include quasi-drugs, and medicaments such as ointment and gel preparation for skin inflammations accompanied by dry skin, for example, chaps, cracks, pruritus and atopy.

The cosmetic composition and external agent can be prepared by a conventional method using the humectant comprising the above specific components and other known components. For example, known oily components, surfactants, humectants, thickeners, antiseptics, pigments, powders, pH adjustors, antioxidants, ultraviolet absorbers, perfumes, dyes and purified water may be appropriately formulated in the humectant comprising the above specific components.

Specific examples of oil agent components and surfactants used in the cosmetic compositions and external agents include polyoxyethylene cetyl ether, polyoxyethylene oleyl ether, polyoxyethylene stearyl ether, polyoxyethylene nonylphenyl ether, polyoxyethylene lauryl ether, polyoxyethylene hydrogenated castor oil, polyoxyethylene sorbitol tetraoleate, sorbitan monooleate, sorbitan tristearate, glyceryl monooleate, glyceryl monostearate, lecithin, lysolecithin, polyglycerin fatty acid ester, and mono-, di-, tri- or tetraester of sucrose and the above fatty acid.

Examples of the thickener include vegetable-based polymers such as gum arabic and gum tragacanth; microorganism-based polymers such as xanthan gum and dextran; starch-based polymers such as carboxymethyl starch; and cellulose-based polymers such as sodium carboxymethylcellulose.

Examples of the powder include inorganic powders such as titanium oxide, magnesium carbonate, mica, and hydroxyapatite; and organic powders such as polyamide powder.

Examples of the antioxidant include BHT, BHA, vitamin A(s), and derivatives and salts thereof; vitamin C(s) and derivatives and salts thereof; and vitamin E(s) and derivatives and salts thereof.

Examples of the ultraviolet absorber include benzophenone derivative, paraaminobenzoic acid derivative, methoxycinnamic acid derivative, and urocanic acid.

Examples of the pigment include colored pigment and pearlescent pigment.

These cosmetic composition components or external agent components are not specifically limited and there can be used those which are commonly as the cosmetic composition components or external agent components.

The cosmetic composition and external agent of the present invention are obtained by appropriately using these components in combination, and mixing, dispersing, emulsifying or dissolving the components with or without heating to give a liquid, paste, gel, cream (including semi-solid) or solid form. The cosmetic composition of the present invention is used by applying directly or indirectly to the skin.

The present invention will be described in detail by the following examples, but the present invention is not limited thereto.

### Preparation Comparative Example 1

In a four-necked flask equipped with a stirrer, a thermometer, a gas blowing tube and a water separator, decaglycerin (75 g, 0.1 mol), stearic acid (185 g, 0.65 mol) and eicosadicarboxylic acid (17 g, 0.05 mol) were charged, and then 0.1% by mass of p-toluenesulfonic acid as a catalyst and 5% by mass of xylene as a refluxing solvent were added. The mixture was esterified by heating to a temperature of 180 to 230°C in a nitrogen gas flow. After 8 hours, the reaction product had an acid value of 0.8 and a decrease in acid value was not confirmed. After the completion of the reaction, xylene was distilled off from the reaction product and the product was decolored with active carbon and then deodorized by blowing steam to obtain 241 g of decaglycerin fatty acid ester eicosane diacid condensate as an ester compound. Each proportion of raw materials of the ester compound, in which half of the hydroxyl groups of decaglycerin remain, is as follows, for example, decaglycerin: 0.1 mol, stearic acid: 0.5 mol, and eicosadicarboxylic acid: 0.05 mol. Since a larger molar amount of stearic acid is charged, the number of the remaining hydroxyl groups of the resulting decaglycerin fatty acid ester eicosane diacid condensate is less than half of the number of hydroxyl groups of decaglycerin, and the resulting decaglycerin fatty acid ester eicosane diacid condensate had an acid value of 0.8 and a hydroxyl value of 70.2.

### Preparation Comparative Example 2

In the same manner as in Preparation Comparative Example 1, decaglycerin (75 g, 0.1 mol), behenic acid (238 g, 0.7 mol) and eicosadicarboxylic acid (17 g, 0.05 mol) were reacted to obtain 305 g of an ester compound. Each proportion of raw materials of the ester compound, in which half of the hydroxyl groups of decaglycerin remain, is as follows, for example, decaglycerin: 0.1 mol, behenic acid: 0.5 mol, and eicosadicarboxylic acid: 0.05 mol. The ester compound obtained by increasing the molar amount of behenic acid, in which the number of the remaining hydroxyl groups is less than half of the number of hydroxyl groups of decaglycerin, had an acid value of 0.8 and a hydroxyl value of 75.7.

### Preparation Comparative Example 3

In the same manner as in Preparation Comparative Example 1, decaglycerin (75 g, 0.1 mol), palmitic acid (179 g, 0.7 mol) and eicosadicarboxylic acid (17 g, 0.1 mol) were reacted to obtain 239 g of an ester compound. Each proportion of raw materials of the ester compound, in which half of the hydroxyl groups of decaglycerin remain, is as follows, for example, decaglycerin: 0.1 mol, palmitic acid: 0.1 mol, and eicosadicarboxylic acid: 0.05 mol. The ester compound obtained by increasing the molar amount of palmitic acid, in which the number of the remaining hydroxyl groups is less than half of the number of hydroxyl groups of decaglycerin, had an acid value of 0.9 and a hydroxyl value of 70.2.

### Preparation Example 1

In the same manner as in Preparation Comparative Example 1, decaglycerin (75 g, 0.1 mol), stearic acid (114 g, 0.4 mol) and eicosadicarboxylic acid (17 g, 0.05 mol) were reacted to obtain 138 g of an ester compound as the component A of the present invention, namely, a decaglycerin fatty acid ester eicosane diacid condensate. Each proportion of raw materials of the ester compound, in which half of the hydroxyl groups of decaglycerin remain, is the same as that described in Preparation Comparative Example 1 and the molar amount of stearic acid is decreased. Therefore, the number of the remaining hydroxyl groups of the resulting decaglycerin fatty acid ester eicosane diacid condensate is more than half of the number of hydroxyl groups of decaglycerin, and the resulting ester compound had an acid value of 0.1 and a hydroxyl value of 470.1.

### Preparation Example 2

In the same manner as in Preparation Comparative Example 1, decaglycerin (75 g, 0.1 mol), behenic acid (34 g, 0.1 mol) and eicosadicarboxylic acid (17 g, 0.05 mol) were reacted to obtain 101 g of an ester compound as the component A of the present invention, namely, a decaglycerin fatty acid ester eicosane diacid condensate. Each proportion of raw materials of the ester compound, in which half of the hydroxyl groups of decaglycerin remain, is the same as that described in Preparation Comparative Example 2, and the decaglycerin fatty acid ester eicosane diacid condensate obtained by decreasing the molar amount of stearic acid, in which more than half of the number of hydroxyl groups of decaglycerin remain, had an acid value of 0.1 and a hydroxyl value of 440.8.

### Preparation Example 3

In the same manner as in Preparation Comparative Example 1, decaglycerin (75 g, 0.1 mol), palmitic acid (26 g, 0.1 mol) and eicosadicarboxylic acid (34 g, 0.05 mol) were reacted to obtain 110 g of an ester compound as the component A of the present invention, namely, a decaglycerin fatty acid ester eicosane diacid condensate. Each proportion of raw materials of the ester compound, in which half of the hydroxyl groups of decaglycerin remain, is the same as that described in Preparation Comparative Example 3, and the decaglycerin fatty acid ester eicosane diacid condensate obtained by decreasing the molar amount of stearic acid, in which more than half of the number of hydroxyl groups of decaglycerin remain, had an acid value of 0.1 and a hydroxyl value of 481.2.

Each of decaglycerin fatty acid ester eicosane diacid condensates obtained in Preparation Comparative Examples 1 to 3 and Preparation Examples 1 to 3, 1,3-butylene glycol (manufactured by DAICEL CHEMICAL INDUSTRIES, LTD.) as a dihydroxy water soluble alcohol, and glycerin as a polyhydroxy alcohol which is trihydroxy or above were mixed according to each formulation shown in Table 1 to Table 3, dissolved by heating to 80°C, and then cooled to room temperature to obtain humectants. A mixing ratio of 1,3-butylene glycol to glycerin was 1:1 (mass ratio). The resulting humectants were stored at 50°C for one month and storage stability were evaluated. The results are shown in Table I to Table 3.

**Table 1: Storage stability of the resulting humectant**

| | Humectant 1 | Humectant 2 | Humectant 3 |
|---|---|---|---|
| Decaglycerin fatty acid ester eicosane diacid condensate | obtained in Preparation Comparative Example 1 | obtained in Preparation Comparative Example 2 | obtained in Preparation Comparative Example 3 |
| | 0.1 g | 0.1 g | 0.1 g |
| 1,3-butylene glycol | 49.95 g | 49.95 g | 49.95 g |
| Glycerin | 49.95 g | 49.95 g | 49.95 g |
| Storage stability (after storage at 50°C for one month) | × | × | × |

| | | | |
|---|---|---|---|
| In Table 1, the symbol × denotes that a deposit was observed. | | | |

**Table 2: Storage stability of the resulting humectant**

| | Humectant 4 | Humectant 5 | Humectant 6 |
|---|---|---|---|
| Decaglycerin fatty acid ester eicosane diacid condensate | obtained in Preparation Example 1 | obtained in Preparation Example 2 | obtained in Preparation Example 3 |
| | 0.1 g | 0.1 g | 0.1 g |
| 1,3-butylene glycol | 49.95 g | 49.95 g | 49.95 g |
| Glycerin | 49.95 g | 49.95 g | 49.95 g |
| Storage stability (after storage at 50°C for one month) | ○ | ○ | ○ |

| | | | |
|---|---|---|---|
| In Table 2, the symbol o denotes that a deposit was not observed. | | | |

**Table 3: Storage stability of the resulting humectant**

| | Humectant 7 | Humectant 8 |
|---|---|---|
| Decaglycerin fatty acid ester eicosane diacid condensate | obtained in Preparation Comparative Example 1 | obtained in Preparation Comparative Example 2 |
| | 0.1 g | 0.1 g |
| 1,3-butylene glycol | 49.95 g | 49.95 g |
| Glycerin | 49.95 g | 49.95 g |
| Storage stability (after storage at 50°C for one month) | × | O |

| | | |
|---|---|---|
| In Table 3, the symbol o denotes that a deposit was not observed and the symbol x denotes that a deposit was observed. | | |

As is apparent from the results shown in Tables 1 to 3, a deposit was observed in the humectant containing a decaglycerin fatty acid ester eicosane diacid condensate, in which less than half of the hydroxyl groups of decaglycerin remain, formulated therein after storage at 50°C for one month, while the humectant containing a decaglycerin fatty acid ester eicosane diacid condensate, in which more than half of the hydroxyl groups of decaglycerin remain, formulated therein is excellent in storage stability regardless of the amount.

The decaglycerin fatty acid ester eicosane diacid condensate used in Comparative Examples 5, 6, 9 and 10, and Examples 1 to 13 was decaglycerin fatty acid ester eicosane diacid condensate as an ester compoud obtained by the method of Preparation Example 2.

### Comparative Examples 1 to 4

According to each formulation shown in Table 4, 100 g of a mixture was obtained, dissolved by heating to 80°C, and then cooled to obtain comparative humectants 1 to 4.

20 g of each of the resulting humectants was heated again to 80°C and mixed with 10 g of purified water at the same temperature while sufficiently stirring. After cooling to room temperature, the resulting solution was charged in a Petri dish and allowed to stand in a room at a temperature of 25°C and a relative humidity of 30%, and then a decrease in moisture content was measured with a lapse of time. The results are shown in Table 4.

**Table 4: Formulation and decrease in moisture content**

| | | Comparative Examples | | | |
|---|---|---|---|---|---|
| | | 1 | 2 | 3 | 4 |
| Components (% by mass) | Decaglycerin fatty acid ester eicosane diacid condensate | 0 | 0 | 0 | 0 |
| | Glycerin | 80 | 50 | 100 | - |
| | 1,3-BG | 20 | 50 | - | 100 |
| Decrease in moisture content (unit: g) | After 6 hours | 0.95 | 1.36 | 1.21 | 0.98 |
| | After 12 hours | 1.59 | 1.99 | 1.98 | 1.65 |
| | After 24 hours | 2.22 | 2.88 | 2.58 | 2.49 |

As is apparent from the results shown in Table 4, all humectants obtained in Comparative Examples 1 to 4 exhibit large decrease in moisture content and the moisture content is likely to be influenced by ambient humidity.

### Comparative Example 5

According to the formulation shown in Table 5, 100 g of a mixture containing a decaglycerin fatty acid ester eicosane diacid condensate and glucose (manufactured by NIHON SHOKUHIN KAKO CO., LTD under the trade name of Nisshoku Medicalose) was obtained, dissolved by heating to 80°C, and then cooled to room temperature to obtain a humectant of Comparative Example 5.

### Comparative Example 6

According to the formulation shown in Table 5, 100 g of a mixture containing a decaglycerin fatty acid ester eicosane diacid condensate, glucose and erythritol (manufactured by NIiKKEN CHEMICALS CO., Ltd. under the trade name of Erythritol) was obtained, dissolved by heating to 80°C, and then cooled to room temperature to obtain a humectant of Comparative Example 6.

20 g of each of the humectants obtained by Comparative Examples 5 and 6 was heated again to 80°C and mixed with 10 g of purified water at the same temperature while sufficiently stirring. After cooling to room temperature, the resulting solution was charged in a Petri dish and allowed to stand in a room at a temperature of 25°C and a relative humidity of 30%, and then a decrease in moisture content was measured with a lapse of time. The results are shown in Table 5.

**Table 5 : Formulation and decrease in moisture content**

| | | Comparative Examples | |
|---|---|---|---|
| | | 5 | 6 |
| Components (% by mass) | Decaglycerin fatty acid ester eicosane diacid condensate | 5 | 5 |
| | Glucose | 95 | 47.5 |
| | Erythritol | 0 | 47.5 |
| Decrease in moisture content (unit: g) | After 6 hours | 1.13 | 1.25 |
| | After 12 hours | 2.14 | 1.66 |
| | After 24 hours | 2.56 | 2.89 |

As is apparent from the results shown in Table 5, all humectants containing no water soluble alcohol as the component B obtained in Comparative Examples 5 and 6 exhibit large decrease in moisture content and the moisture content is likely to be influenced by ambient humidity.

### Examples 1 to 3

According to each formulation shown in Table 6, 100 g of a mixture containing a decaglycerin fatty acid ester eicosane diacid condensate, 1,3-BG and glycerin was obtained, dissolved by heating to 80°C, and then cooled to room temperature to obtain gel humectants 1 to 3 of these examples.

20 g of each of the resulting humectants was heated again to 80°C and mixed with 10 g of purified water at the same temperature while sufficiently stirring. After cooling to room temperature, the resulting solution was charged in a Petri dish and allowed to stand in a room at a temperature of 25°C and a relative humidity of 30%, and then a decrease in moisture content was measured with a lapse of time. The results are shown in Table 6.

**Table 6: Formulation and decrease in moisture content**

| | | Examples | | |
|---|---|---|---|---|
| | | 1 | 2 | 3 |
| Components (% by mass) | Decaglycerin fatty acid ester eicosane diacid condensate | 5 | 5 | 5 |
| | Glycerin | 76 | 47.5 | 24 |
| | 1,3-BG | 19 | 47.5 | 71 |
| Decrease in moisture content (unit: g) | After 6 hours | 0.33 | 0.38 | 0.44 |
| | After 12 hours | 0.55 | 0.73 | 0.74 |
| | After 24 hours | 0.98 | 1.02 | 0.11 |

As is apparent from the results shown in Table 6, all gel humectants of Examples 1 to 3 exhibit small decrease in moisture content and are excellent in moisture retaining properties, and also these gel humectants are suited for use as the humectant and are excellent in stability. Comparing the evaluation results of the Examples shown in Table 6 with the evaluation results of the Comparative Examples shown in Tables 4 and 5, the humectants of Examples 1 to 3 are excellent in moisture retaining properties and stability as compared with the humectants of the Comparative Examples.

### Examples 4 to 7

According to each formulation shown in Table 7, 100 g of a mixture containing a decaglycerin fatty acid ester eicosane diacid condensate, 1,3-BG and glycerin was obtained, dissolved by heating to 80°C, and then cooled to room temperature to obtain humectants 4 to 7, that is, viscous humectants of Example 4 and 5 and gel humectants of Examples 6 and 7.

20 g of each of the resulting humectants was heated again to 80°C and mixed with 10 g of purified water at the same temperature while sufficiently stirring. After cooling to room temperature, the resulting solution was charged in a Petri dish and allowed to stand in a room at a temperature of 25°C and a relative humidity of 30%, and then a decrease in moisture content was measured with a lapse of time. The results are shown in Table 7.

**Table 7: Formulation and decrease in moisture content**

| | | Examples | | | |
|---|---|---|---|---|---|
| | | 4 | 5 | 6 | 7 |
| Components (% by mass) | Decaglycerin fatty acid ester eicosane diacid condensate | 0.1 | 1.0 | 10 | 20 |
| | Glycerin | 49.95 | 49.5 | 45 | 40 |
| | 1,3-BG | 49.95 | 49.5 | 45 | 40 |
| Decrease in moisture content (unit: g) | After 6 hours | 1.01 | 0.66 | 0.29 | 0.15 |
| | After 12 hours | 1.28 | 1.05 | 0.55 | 0.28 |
| | After 24 hours | 1.58 | 1.44 | 0.95 | 0.33 |

As is apparent from the results shown in Table 7, all gel humectants of Examples 4 to 7 exhibit small decrease in moisture content as compared with the above Comparative Examples and are suited for use as the humectant.

### Comparative Examples 7 and 8

According to each formulation shown in Table 8, 100 g of a mixture containing 1,3-BG and glycerin was obtained, dissolved by heating to 80°C, and then cooled to room temperature to obtain humectants of Comparative Examples 7 and 8.

### Examples 8 and 9

According to each formulation shown in Table 8, 100 g of a mixture containing a decaglycerin fatty acid ester eicosane diacid condensate, 1,3-BG and glycerin was obtained, dissolved by heating to 80°C, and then cooled to room temperature to obtain viscous humectants of Example 8 and 9. A mixing ratio of 1,3-BG to glycerin was 1:1 (mass ratio).

20 g of each of the resulting humectants was heated again to 80°C and mixed with 5 g of purified water at 80°C (Comparative Example 7 and Example 8) or mixed with 20 g of purified water at 80°C (Comparative Examples 8 and Example 9) while sufficiently stirring. After cooling to room temperature, the resulting solution was charged in a Petri dish and allowed to stand in a room at a temperature of 25°C and a relative humidity of 30%, and then a decrease in moisture content was measured with a lapse of time. The results are shown in Table 8.

**Table 8: Formulation and decrease in moisture content**

| | | Comparative Examples | | Examples | |
|---|---|---|---|---|---|
| | | 7 | 8 | 8 | 9 |
| Components (% by mass) | Decaglycerin fatty acid ester eicosane diacid condensate | 0 | 0 | 5 | 5 |
| | Glycerin | 50.0 | 50.0 | 47.5 | 47.5 |
| | 1,3-BG | 50.0 | 50.0 | 47.5 | 47.5 |
| Amount of purified water added to 20 g of each of humectant (g) | | 5 | 20 | 5 | 20 |
| Decrease in moisture content (g) | After 6 hours | 0.48 | 1.45 | 0.18 | 0.74 |
| | After 12 hours | 0.89 | 2.78 | 0.30 | 1.39 |
| | After 24 hours | 1.25 | 4.78 | 0.46 | 2.23 |

As is apparent from the results shown in Table 8, the humectants of Examples 8 and 9 exhibit small decrease in moisture content as compared with the humectants containing no decaglycerin fatty acid ester eicosane diacid condensate as the component added therein of Comparative Examples 7 and 8 even if the humectants contain water added therein, and thus the resulting humectants are suited for use as the humectant. The same results were obtained even if the amount of water varies.

### Comparative Example 9 and 10 and Example 10 to 13

According to each formulation shown in Table 9, the respective components were mixed, heated with stirring at 80°C, and ten cooled to obtain humectants of the

### Comparative Examples and Examples.

To evaluate stability of the resulting humectants, the humectants were allowed to stand at room temperature (20°C) and low temperature (5°C) for 24 hours and the condition was observed. The results are shown in Table 9.

**Table 9: Formulation and stability (unit: % by mass)**

| Components | | Comparative Examples | | Examples | | | |
|---|---|---|---|---|---|---|---|
| | | 9 | 10 | 10 | 11 | 12 | 13 |
| Decaglycerin fatty acid ester eicosane diacid condensate | | 10 | 20 | 10 | 5 | 25 | 30 |
| Glycerin | | 30 | 40 | 30 | 20 | | 40 |
| Sorbitol 70¹⁾ | | | | | | 30 | |
| 1,3-BG | | | | 30 | 40 | 30 | 30 |
| Ethanol | | 30 | 30 | | | | |
| Water | | 30 | 10 | 30 | 35 | 15 | |
| Stability²⁾ | 20°C | Δ | ○ | ○ | ○ | ○ | ○ |
| | 5°C | Δ | Δ | ○ | ○ | ○ | ○ |

| | | | | | | | |
|---|---|---|---|---|---|---|---|
| In Table 9, the numeral reference 1) denotes an aqueous solution containing 70% by mass of sorbitol, while the numeral reference 2) denotes evaluation criteria of stability, in which the symbol "o" denotes the case where no deposit is observed and the symbol "Δ" denotes the case where a deposit is observed. | | | | | | | |

As is apparent from the results shown in Table 9, the viscous humectants of Examples 10 and 11, and the gel humectants of Examples 12 and 13 are excellent in stability at room temperature and low temperature.

Then, 50% by mass of purified water was added to 100% by mass of the humectants obtained in Comparative Examples 2 and 4 and 100% by mass of the humectants obtained in Examples 3 and 5 to obtain humectants containing water. First, the viscosity at 25°C of the resulting humectants containing water was measured. Also the viscosity was measured after storing the resulting humectants at 50°C for one month using a BL type viscometer. Furthermore, stability of the humectants was visually evaluated by confirming whether or not separation is observed in the humectant stored at 50°C for one month. The results are shown in Table 10.

**Table 10: Physical properties and stability**

| | | Viscosity (mPa·s) | Stability |
|---|---|---|---|
| Comparative Examples | 2 | 20 (25°C) | - |
| | 2 | 15 (50°C) | × |
| | 4 | 17 (25°C) | - |
| | 4 | 14 (50°C) | × |
| Examples | 3 | 3,500 (25°C) | - |
| | 3 | 3,400 (50°C) | ○ |
| | 5 | 3,250 (25°C) | - |
| | 5 | 3,000 (50°C) | ○ |

| | | | |
|---|---|---|---|
| In Table 10, the symbol "o" denotes the case where no separation is observed and the symbol "x" denotes the case where separation is observed, and the symbol "-" denotes that the measurement was not conducted. | | | |

As is apparent from the results shown in Table 10, the mixtures obtained by adding 50% by mass of purified water to the humectants of Examples 3 and 5 can retain high viscosity at normal temperature (25°C) and high temperature (50°C). Also it was confirmed that separation of the humectants was not observed even when stored at 50°C for one month and therefore the humectants are excellent in stability at high temperature.

### Example 14

According to each formulation shown in Tables 11 and 12, emulsion type cosmetic bases containing an oil-in-water type humectant were prepared in the following manner.

Method for preparation of samples: A first component was mixed with a second component while stirring at a temperature of 80°C and, after gradually adding a third component, purified water was further added and the mixture was cooled to 20°C.

The resulting cosmetic bases were evaluated and measured in the following manner. The results are shown in Tables 11 and 12.

Method for measurement of conductance of skin surface: After cleansing the upper arm inside part of healthy female using ethanol, 0.02 g of the resulting cosmetic base was applied on the skin surface within a region having a radius of 3 cm. After 60 minutes, the conductance of the part was measured in a room at a temperature of 19 to 21°C and a relative humidity of 30 to 40% using a high-frequency conductometry (manufactured by IBS Co. under the trade name of SKICON-200). The measured values of three subjects were averaged.

Evaluation of moisture retaining ability: The measurement results of the conductance of the skin surface were evaluated according to the following four-rank criteria, ⓪ denotes that remarkable moisture retaining effect is exerted, o denotes that ordinary moisture retaining effect is exerted, Δ denotes that less moisture retaining effect is exerted, and x denotes that no moisture retaining effect is exerted.

Evaluation of stability: After storing the resulting cosmetic bases at 50°C for one month, stability was visually evaluated by confirming whether or not separation is observed. In the table, the symbol o denotes the case where no deposit is observed and the symbol Δ denotes the case where with a deposit is observed.

**Table 11: Composition and evaluation of cosmetic base (Amount of each component: % by weight)**

| | | Comparative products | | Products of the present invention | | | |
|---|---|---|---|---|---|---|---|
| Sample No. | | 5 | 6 | 1 | 2 | 3 | 4 |
| First component | Decaglycerin fatty acid ester eicosane diacid condensate¹⁾ | 5 | 5 | 5 | 5 | 5 | 5 |
| Second component | Glycerin | 20 | | 10 | 5 | 15 | 10 |
| | 1,3-BG²⁾ | | | 10 | 15 | 5 | |
| | Propylene glycol | | | | | | 10 |
| Third component | Olive oil | 10 | 10 | 10 | 10 | 10 | 10 |
| | Cetanol | 2 | 2 | 2 | 2 | 2 | 2 |
| Purified water | | 63 | 83 | 83 | 63 | 63 | 63 |
| Conductance of skin surface (µm) | | 100 | 105 | 251 | 223 | 240 | 168 |
| Evaluation of moisture retaining ability | | Δ | Δ | ⓪ | ⓪ | ⓪ | ○ |
| Evaluation of stability | | Δ | Δ | ○ | ○ | ○ | ○ |

| | | | | | | | |
|---|---|---|---|---|---|---|---|
| 1) manufactured by Nisshin Oil Mills Ltd.: Nomcort HK-P 2) 1,3-butylene glycol: manufactured by DAICEL CHEMICAL INDUSTRIES, LTD. | | | | | | | |

**Table 12: Composition and evaluation of cosmetic base (Amount of each component: % by weight)**

| | | Comparative products | | | | | Products of the present invention | | | |
|---|---|---|---|---|---|---|---|---|---|---|
| Sample No. | | 12 | 13 | 14 | 15 | 16 | 8 | 9 | 10 | 11 |
| First component | Decaglycerin fatty acid ester eicosane diacid condensate¹⁾ | | | | | | 0.5 | 1 | 2 | 10 |
| | Tween²⁾ | 1 | 1 | 1 | 1 | 1 | | | | |
| | Span 80³⁾ | 1 | 1 | 1 | 1 | 1 | | | | |
| Second component | Glycerin | 10 | | | | | 10 | 10 | 10 | 10 |
| | 1,3-BG⁴⁾ | 10 | 20 | 10 | 10 | 10 | 10 | 10 | 10 | 10 |
| | 3-M-1,3-BG | | | 10 | | | | | | |
| | Propylene glycol | | | | 10 | | | | | |
| Third component | Olive oil | 10 | 10 | 10 | 10 | 10 | 10 | 10 | 10 | 10 |
| | Cetanol | 2 | 2 | 2 | 2 | 2 | 2 | 2 | 2 | 2 |
| Purified water | | 66 | 66 | 66 | 66 | 66 | 68 | 67 | 66 | 58 |
| Conductance of skin surface (µm) | | 90 | 108 | 88 | 94 | 110 | 151 | 165 | 210 | 255 |
| Evaluation of moisture retaining ability | | Δ | Δ | × | × | Δ | ○ | ○ | ⓪ | ⓪ |
| Evaluation of stability | | Δ | Δ | Δ | Δ | Δ | ○ | ○ | ○ | ○ |

| | | | | | | | | | | |
|---|---|---|---|---|---|---|---|---|---|---|
| 1) manufactured by Nisshin Oil Mills Ltd.: Nomcort HK-P 2) ethylene oxide added sorbitan monooleate: manufactured by TOKYO KASEI KOGYO CO., LTD. 3) sorbitan monooleate: manufactured by TOKYO KASEI KOGYO CO., LTD. 4) 1,3-BG: manufactured by DAICEL CHEMICAL INDUSTRIES, LTD. | | | | | | | | | | |

As is apparent from the results shown in Tables 11 and 12, the samples containing no dihydroxy water soluble alcohol (samples Nos. 5 and 6) and the samples using no decaglycerin fatty acid ester eicosane diacid condensate in combination (samples Nos. 12 to 16) exhibit small conductance and exert poor moisture retaining effect. To the contrary, samples (samples Nos. 1 to 4 and 8 to 11) exhibit large conductance of the skin surface and exert excellent skin moisture retaining effect. Also it was confirmed that the samples are excellent in stability.

### Example 15

According to each base formulation shown in Tables 13 and 14, beauty wash type cosmetic bases containing an oil-in-water type humectant were prepared in the following manner.

Method for preparation of samples: A first component was mixed with a second component while stirring at a temperature of 80°C and, after gradually adding a third component, purified water containing a pH adjustor added therein was gradually added and the mixture was cooled to 20°C. The amount of purified water was indicated by a value including a trace amount the pH adjustor. Although the amount of the pH adjustor added was indicated by the trace amount, it means a minimum amount enough to maintain the pH of the resulting samples within a range from about 6.5 to 7.0.

The resulting cosmetic bases were evaluated and measured in the same manner as in Example 14. The results are shown in Tables 13 and 14.

**Table 13: Composition and evaluation of cosmetic base (Amount of each component: % by mass)**

| | | Comparative products | | Products of the present invention | | | |
|---|---|---|---|---|---|---|---|
| Sample No. | | 5 | 6 | 1 | 2 | 3 | 4 |
| First component | Decaglycerin fatty acid ester eicosane diacid condensate¹⁾ | 5 | 5 | 5 | 5 | 5 | 5 |
| Second component | Glycerin | 20 | 10 | 10 | 5 | 15 | 10 |
| | 1,3-BG²⁾ | | | 10 | 15 | 5 | |
| | propylene glycol | | | | | | 10 |
| Third component | Olive oil | 10 | 10 | 10 | 10 | 10 | 10 |
| | Cetanol | 2 | 2 | 2 | 2 | 2 | 2 |
| pH adjustor | | trace amount | trace amount | trace amount | trace amount | trace amount | trace amount |
| Purified water | | 63 | 73 | 63 | 63 | 63 | 63 |
| Conductance of skin surface (µm) | | 85 | 80 | 211 | 210 | 216 | 201 |
| Evaluation of moisture retaining ability | | × | × | ⓪ | ⓪ | ⓪ | ⓪ |
| Evaluation of stability | | Δ | Δ | ○ | ○ | ○ | ○ |

| | | | | | | | |
|---|---|---|---|---|---|---|---|
| 1) manufactured by Nisshin Oil Mills Ltd.: Nomcort HK-P 2) 1,3-BG : manufactured by DAICEL CHEMICAL INDUSTRIES, LTD. | | | | | | | |

**Table 14: Composition and evaluation of cosmetic base (Amount of each component: % by mass)**

| | | Comparative products | | | | | Products of the present invention | | | |
|---|---|---|---|---|---|---|---|---|---|---|
| Sample No. | | 7 | 8 | 9 | 10 | 11 | 12 | 13 | 14 | 15 |
| First | Decaglycerin fatty acid ester eicosane diacid condensate¹⁾ | | | | | | 0.5 | 1 | 2 | 10 |
| Second component | Glycerin | 10 | | | | | 10 | 10 | 10 | 10 |
| | 1,3-BG²⁾ | 10 | 20 | 10 | 10 | 10 | 10 | 10 | 10 | 10 |
| | 3-M-1,3-BG | | | 10 | | | | | | |
| | Propylene glycol | | | | 10 | | | | | |
| Third component | Olive oil | 10 | 10 | 10 | 10 | 10 | 10 | 10 | 10 | 10 |
| | Cetanol | 2 | 2 | 2 | 2 | 2 | 2 | 2 | 2 | 2 |
| pH adjustor | | trace amount | trace amount | trace amount | trace amount | trace amount | trace amount | trace amount | trace amount | trace amount |
| Purified water | | 66 | 66 | 66 | 66 | 66 | 68 | 67 | 66 | 58 |
| Conductance of skin surface (µm) | | 54 | 50 | 55 | 49 | 59 | 154 | 168 | 179 | 199 |
| Evaluation of moisture retaining ability | | × | × | × | × | × | ⓪ | ⓪ | ⓪ | ⓪ |
| Evaluation of stability | | Δ | Δ | Δ | Δ | Δ | ○ | ○ | ○ | ○ |

| | | | | | | | | | | |
|---|---|---|---|---|---|---|---|---|---|---|
| 1) manufactured by Nisshin Oil Mills Ltd.: Nomcort HK-P 2) 1,3-BG: manufactured by DAICEL CHEMICAL INDUSTRIES, LTD. | | | | | | | | | | |

As is apparent from the results shown in Tables 13 and 14, the samples containing no dihydroxy water soluble alcohol (samples Nos. 5 and 6) and the samples using no decaglycerin fatty acid ester eicosane diacid condensate in combination (samples Nos. 7 to 11) exhibit small conductance and exert poor moisture retaining effect. To the contrary, samples (samples Nos. 1 to 4 and 12 to 15) exhibit large conductance of the skin surface and exert excellent skin moisture retaining effect. Also it was confirmed that the samples are excellent in stability.

### Example 16

According to the base formulation shown in Table 15, cleansing composition type cosmetic bases containing a viscous humectant were prepared in the following manner.

Method for preparation of samples: A first component was mixed with a second component while stirring at a temperature of 80°C and, after gradually adding a third component, purified water was further added to emulsify and the mixture was cooled to 20°C.

The resulting cosmetic bases were evaluated and measured in the following manner. The results are shown in Table 15.

Method for measurement of conductance of skin surface: After cleansing the upper arm inside part of healthy female using ethanol, 0.02 g of the resulting cosmetic base was applied on the skin surface within a region having a radius of 3 cm. After massaging, the skin surface was washed with water and then dewatered. After 60 minutes, the conductance of the part was measured in a room at a temperature of 19 to 21°C and a relative humidity of 30 to 40% using a high-frequency conductometry (manufactured by IBS Co. under the trade name of SKICON-200). The measured values of three subjects were averaged.

Evaluation of moisture retaining ability: The measurement results of the conductance of the skin surface were evaluated according to the following four-rank criteria, ⓪ denotes that remarkable moisture retaining effect is exerted, o denotes that ordinary moisture retaining effect is exerted, Δ denotes that less moisture retaining effect is exerted, and x denotes that no moisture retaining effect is exerted.

**Table 15: Composition and evaluation of cosmetic base (Amount of each component: % by weight)**

| | | Comparative products | | | Products of the present invention | | |
|---|---|---|---|---|---|---|---|
| Sample No. | | 4 | 5 | 6 | 1 | 2 | 3 |
| First component | Decaglycerin fatty acid ester eicosane diacid condensate¹⁾ | | | | 2 | 2 | 2 |
| Second component | Sorbitol70²⁾ | | | | 20 | 10 | 30 |
| | 1,3-BG³⁾ | | | | 20 | 30 | 10 |
| Third component | Isopropyl myristate | | | | 58 | 58 | 58 |
| Purified water | | 100 | | | | | |
| 10% aqueous potassium laurate solution | | | 100 | | | | |
| Cleansing oil⁴⁾ | | | | 100 | | | |
| Conductance of skin surface (µm) | | 35 | 30 | 29 | 102 | 104 | 101 |
| Evaluation of moisture retaining ability | | × | × | × | ○ | ○ | ○ |

| | | | | | | | |
|---|---|---|---|---|---|---|---|
| 1) manufactured by Nisshin Oil Mills Ltd.: Nomcort HK-P 2) aqueous 70 wt% sorbitol solution 3) 1,3-BG: manufactured by DAICEL CHEMICAL INDUSTRIES, LTD. 4) composition: Tween 80: 5% by mass, Span 80: 5% by mass, liquid paraffin: 40% by mass, and isopropyl myristate: 50% by mass | | | | | | | |

As is apparent from the results shown in Table 15, the samples (samples Nos. 4 to 6) containing water, an aqueous potassium laurate solution, and a conventional cleansing oil, in addition to the first to third components exhibit small conductance of the skin surface after subjecting to a cleansing treatment and therefore exert poor moisture retaining effect. To the contrary, the samples of the Examples (samples Nos. 1 to 3) exhibit large conductance of the skin surface after subjecting to a cleansing treatment and therefore exert excellent skin moisture retaining effect. Also it was confirmed that the samples are excellent in stability.

### Example 17 [Trial preparation of cream]

Using the raw material components (1) to (10) shown in Table 16, an oil-in-water type moisture cream was prepared in the following manner. First, the components (1) to (3) were mixed with heating to 80°C to obtain a uniformly dissolved humectant. Separately, the components (4) to (9) were mixed with heating to 85°C to obtain an oil phase. While stirring at a temperature of 80°C, the oil phase was gradually added to the humectant to obtain a viscous humectant. The component (10) heated to 80°C was poured into the resulting viscous humectant, followed by stirring to form an emulsion, which was cooled to room temperature to obtain an oil-in-water type moisture cream.

The cream obtained in this example gave excellent moist touch to female with dry skin. The cream exhibited stable physical properties without causing any separation of the components nor any deposition in a room (temperature: 20 to 25°C, humidity: 40 to 60%, the conditions are the same in examples described hereinafter) for one year.

**Table 16: Composition of oil-in-water type moisture cream (% by mass)**

| | |
|---|---|
| (1) Sorbitol solution (70%) | 10.0 |
| (2) Decaglycerin fatty acid ester eicosane diacid condensate | 4.0 |
| (3) 1,3-BG | 5.0 |
| (4) Stearyl alcohol | 2.0 |
| (5) Microcrystalline wax | 2.0 |
| (6) Squalane | 5.0 |
| (7) Glyceryl trioctanoate | 10.0 |
| (8) Octyldodecyl myristate | 5.0 |
| (9) Methyl paraoxybenzoate | 0.3 |
| (10) Purified water | balance |
| Total | 100.0 |

### Example 18 [Trial preparation of emulsion]

Using the raw material components (1) to (11) shown in Table 17, an oil-in-water type emulsion was prepared in the following manner. First, the components (1) to (3) were mixed with heating to 80°C to obtain a uniformly dissolved humectant. Separately, the components (4) to (10) were mixed with heating to 85°C to obtain an oil phase. While stirring at a temperature of 80°C, the oil phase was gradually added to the humectant to obtain a viscous humectant. The component (11) heated to 80°C was poured into the resulting viscous humectant, followed by stirring to form an emulsion, which was cooled to room temperature to obtain an oil-in-water type emulsion.

The emulsion obtained in this example caused no discomfort during use and retained sufficient moist touch after one day has passed since application, and also the emulsion was stable in a room for one year.

**Table 17: Composition of oil-in-water type emulsion (% by mass).**

| | |
|---|---|
| (1) Glycerin | 8.0 |
| (2) Decaglycerin fatty acid ester eicosane diacid condensate | 4.0 |
| (3) 1,3-BG | 10.0 |
| (4) Cetanol | 1.0 |
| (5) Candelilla wax | 1.0 |
| (6) Liquid paraffin | 5.0 |
| (7) Isopropyl palmitate | 5.0 |
| (8) Isononyl nonanoate | 5.0 |
| (9) Methylphenylpolysiloxane | 1.0 |
| (10) Ethyl paraoxybenzoate | 0.1 |
| (11) Purified water | balance |
| Total | 100.0 |

### Example 19 [Trial preparation 1 of beauty wash]

Using the raw material components (1) to (8) shown in Table 18, a solubilized beauty wash 1 was prepared in the following manner. First, the components (1) to (6) were mixed with heating to 80°C to obtain a uniformly dissolved humectant. Separately, the components (7) and (8) were mixed with heating to 80°C to obtain an aqueous solution. While stirring at a temperature of 80°C, the aqueous solution was gradually added to the humectant to obtain an isotropic solubilized solution, which was cooled to room temperature to obtain a beauty wash.

The beauty wash obtained in this example gave good tactile sensation during use and retained moist touch after one day has passed since application, and also the emulsion was stable in a room for one year.

**Table 18: Composition of beauty wash 1 (% by mass)**

| | |
|---|---|
| (1) Glycerin | 10.0 |
| (2) Decaglycerin fatty acid ester eicosane diacid condensate | 4.0 |
| (3) 1,3-BG | 12.0 |
| (4) Purified water (i) | 1.0 |
| (5) Squalane | 0.1 |
| (6) Ethyl paraoxybenzoate | 0.1 |
| (7) Queen's seed | 0.1 |
| (8) Purified water (ii) | balance |
| Total | 100.0 |

### Example 20 [Trial preparation 2 of beauty wash]

Using the raw material components (1) to (8) shown in Table 19, a beauty wash 2 was prepared in the following manner. First, the components (1) to (6) were mixed with heating to 80°C to obtain a uniformly dissolved humectant. Separately, the components (7) and (8) were mixed with heating to 80°C to obtain an aqueous solution. While stirring at a temperature of 80°C, the aqueous solution was gradually added to the humectant to obtain a white turbid emulsion, which was cooled to room temperature to obtain a beauty wash 2.

The beauty wash 2 obtained in this example gave good tactile sensation during use and retained moist touch after one day has passed since application, and also the emulsion was stable in a room for one year.

**Table 19: Composition of beauty wash 2 (% by mass)**

| | |
|---|---|
| (1) Glycerin | 5.0 |
| (2) Diglycerin | 5.0 |
| (3) Decaglycerin fatty acid ester eicosane diacid condensate | 4.0 |
| (4) 1,3-BG | 10.0 |
| (5) Dicaprylic acid neopentyl glycol ester | 1.0 |
| (6) Ethyl paraoxybenzoate | 0.1 |
| (7) Sodium hyaluronate | 0.1 |
| (8) Purified water | balance |
| Total | 100.0 |

### Example 21 [Trial preparation of cleansing gel]

Using the raw material components (1) to (11) shown in Table 20, a cleansing gel was prepared in the following manner. First, the components (1) to (5) were mixed with heating to 80°C to obtain a uniformly dissolved humectant. Separately, the components (6) and (11) were mixed with heating to 80°C to obtain an oil phase. While stirring at a temperature of 80°C, the oil phase was gradually added to the humectant to obtain a gel humectant, which was cooled to room temperature to obtain a cleansing gel.

The cleansing gel obtained in this example was excellent in cleansing properties and washing-away properties with water, and retained moist touch of the skin after the washing-away, and also gave less stretched touch.

**Table 20: Composition of cleansing gel (% by mass)**

| | |
|---|---|
| (1) Glycerin | 25.0 |
| (2) Decaglycerin fatty acid ester eicosane diacid condensate | 4.0 |
| (3) 1,3-BG | 10.0 |
| (4) Purified water | 2.0 |
| (5) Chamomile extract | 1.0 |
| (6) Squalane | 10.0 |
| (7) Liquid paraffin | 10.0 |
| (8) Glyceryl trioctanoate | 18.0 |
| (9) Octyldodecyl myristate | 5.0 |
| (10) Isooctyl palmitate | 10.0 |
| (11) Jojoba oil | 5.0 |
| Total | 100.0 |

### Example 22 [Trial preparation of moisture gel]

Using the raw material components (1) to (7) shown in Table 21, a moisture gel was prepared in the following manner. First, the components (1) to (3) were mixed with heating to 80°C to obtain a uniformly dissolved humectant. The resulting humectant was mixed with the component (4) heated to 80°C and then mixed with a mixed solution of the components (5) to (7) to obtain a transparent moisture gel.

The moisture gel obtained in this example was excellent in stability and retained excellent moist touch after one day has passed since application.

**Table 21: Composition of moisture gel (% by mass)**

| | |
|---|---|
| (1) Glycerin | 10.0 |
| (2) Decaglycerin fatty acid ester eicosane diacid condensate | 4.0 |
| (3) 1,3-BG | 10.0 |
| (4) 1% aqueous carboxyvinyl polymer solution | 40.0 |
| (5) 1% aqueous potassium hydroxide solution | 10.0 |
| (6) 10% aqueous sodium hyaluronate solution | 1.0 |
| (7) Purified water | balance |
| Total | 100.0 |

### Example 23 [Trial preparation of pack]

Using the raw material components (1) to (10) shown in Table 22, a pack was prepared in the following manner. First, the components (1) to (4) were mixed with heating to 80°C to obtain a uniformly dissolved humectant. The components (5) and (6) were dispersed in the component (10) and, after dispersing the resulting humectant in the solution, the components (7) to (9) were finally dispersed to obtain a muddy pack.

The pack obtained in this example was excellent in soft touch and wet touch of the skin after use.

**Table 22: Composition of pack (% by mass)**

| | |
|---|---|
| (1) Sorbitol (70%) | 10.0 |
| (2) Triglycerin | 10.0 |
| (3) Decaglycerin fatty acid ester eicosane diacid condensate | 4.0 |
| (4) 1,3-BG | 10.0 |
| (5) Montmorillonite | 1.0 |
| (6) Ethanol | 5.0 |
| (7) Titanium oxide | 5.0 |
| (8) Kaolin | 10.0 |
| (9) Talc | 5.0 |
| (10) Purified water | balance |
| Total | 100.0 |

### Example 24 [Trial preparation of emulsion foundation]

Using the raw material components (1) to (14) shown in Table 23, an emulsion foundation was prepared in the following manner. First, the components (1) to (4) were mixed with heating to 80°C to obtain a uniformly dissolved humectant. Separately, the components (9) to (13) were mixed with heating to 85°C to obtain an oil phase. While stirring at a temperature of 80°C, the oil phase was gradually added to the humectant to obtain a viscous humectant. The component (14) heated to 80°C was poured into the viscous humectant, followed by stirring to form an emulsion. The components (5) to (8) were dispersed in the emulsion and the mixture was cooled to room temperature to obtain an emulsion foundation.

The emulsion foundation obtained in this example gave extremely less dry touch of the skin after applying the emulsion foundation to the skin and wiping up it.

**Table 23: Composition of emulsion foundation (% by mass)**

| | |
|---|---|
| (1) Glycerin | 10.0 |
| (2) Decaglycerin fatty acid ester eicosane diacid condensate | 4.0 |
| (3) 1,3-BG | 10.0 |
| (4) Purified water (i) | 5.0 |
| (5) Talc | 3.0 |
| (6) Titanium oxide | 5.0 |
| (7) Red iron oxide | 0.5 |
| (8) Yellow iron oxide | 1.0 |
| (9) Liquid paraffin | 5.0 |
| (10) Cetyl octanoate | 10.0 |
| (11) Octyldodecyl lactate | 3.0 |
| (12) Lanoline | 2.0 |
| (13) Cetanol | 2.0 |
| (14) Purified water (ii) | balance |
| Total | 100.0 |

### Example 25 [Trial preparation of bath salt]

Using the raw material components (1) to (9) shown in Table 24, a bath salt was prepared in the following manner. First, the components (1) to (6) were mixed with heating to 80°C to obtain a uniformly dissolved humectant. Separately, the components (7) to (9) were mixed with heating to 80°C to obtain an oil phase. While stirring at a temperature of 80°C, the oil phase was gradually added to the humectant to obtain a gel humectant, which was cooled to room temperature to obtain a white turbid bath salt.

The bath salt obtained in this example retained sufficient moist touch of the skin after taking a bath, and gave no sticky touch.

**Table 24: Composition of bath salt (% by mass)**

| | |
|---|---|
| (1) Glycerin | 30.0 |
| (2) Decaglycerin fatty acid ester eicosane diacid condensate | 4.0 |
| (3) 1,3-BG | 10.0 |
| (4) Chamomile extract | 1.0 |
| (5) Japanese angelica root extract | 1.0 |
| (6) Iris extract | 1.0 |
| (7) Liquid paraffin | 10.0 |
| (8) Glyceryl trioctanoate | 38.0 |
| (9) Methylphenylpolysiloxane | 5.0 |
| Total | 100.0 |

### Example 26 [Ointment]

Using the raw material components (1) to (12) shown in Table 25, an ointment was prepared in the following manner. First, the components (1) to (4) were mixed with heating to 80°C to obtain a uniformly dissolved humectant. Separately, the components (5) to (11) were mixed with heating to 85°C to obtain an oil phase. While stirring at a temperature of 80°C, the oil phase was gradually added to the humectant to obtain a viscous humectant. The component (12) heated to 80°C was poured into the viscous humectant, followed by stirring to form an emulsion, which was cooled to room temperature to obtain a hydrophilic ointment.

The ointment obtained in this example was excellent in affinity with the skin and gave moist touch thereto, and was excellent in stability without causing any separation of the components in the room for one year.

**Table 25: Composition of ointment (% by mass)**

| | |
|---|---|
| (1) Glycerin | 10.0 |
| (2) Decaglycerin fatty acid ester eicosane diacid condensate | 4.0 |
| (3) 1,3-BG | 15.0 |
| (4) Purified water (i) | 5.0 |
| (5) Glyceride monostearate | 1.0 |
| (6) Stearyl alcohol | 5.0 |
| (7) Vaseline | 10.0 |
| (8) Isopropyl myristate | 10.0 |
| (9) Propyl paraoxybenzoate | 0.1 |
| (10) Methyl paraoxybenzoate | 0.1 |
| (11) Glycyrrhetic acid | 0.3 |
| (12) Purified water (ii) | balance |
| Total | 100.0 |

### Example 27 [Gel]

Using the raw material components (1) to (9) shown in Table 26, a gel was prepared in the following manner. First, the components (1) to (3) were mixed with heating to 80°C to obtain a uniformly dissolved humectant. A mixed solution of the components (4) to (6) heated to 80°C was mixed with the resulting humectant and cooled, and then the components (7) to (9) were added to obtain a gel.

The gel obtained in this example was excellent in tactile sensation during use and gave moist touch and wet touch to the skin, and was excellent in stability without causing any separation of the components nor any deposition nor any odor in the room for one year.

**Table 26: Composition of gel (% by mass)**

| | |
|---|---|
| (1) Glycerin | 20.0 |
| (2) Decaglycerin fatty acid ester eicosane diacid condensate | 10.0 |
| (3) 1,3-BG | 20.0 |
| (4) Polyethylene glycol 400 | 20.0 |
| (5) 10% aqueous carboxyvinyl polymer solution | 4.0 |
| (6) 10% aqueous sodium hydroxide solution | 1.0 |
| (7) Ethanol | 2.0 |
| (8) Dipotassium glycyrrhizinate | 0.3 |
| (9) Purified water | balance |
| Total | 100.0 |

### INDUSTRIAL APPLICABILITY

The humectant of the present invention is excellent in moisture retaining properties and stability at high temperature. Also the cosmetic composition or external agent containing the humectant of the present invention is excellent in moisture retaining properties and stability at high temperature and is also excellent in tactile sensation during and after use. Furthermore, since the humectant of the present invention can increase the viscosity of an emulsion without adding thickeners, a viscous or gel cosmetic composition or external agent can be obtained by using the humectant of the present invention. Since the humectant of the present invention can take an oil-in-water emulsion form or a solubilized form, an oil-in-water emulsion or solubilized cosmetic composition or external agent can be obtained by using the humectant of the present invention.

## Claims

1. A humectant comprising :
■ a component A: an ester compound comprising a condensate of glycerin having polymerization degree of 5 or more, a straight chain saturated fatty acid having 16-28 carbons, and an aliphatic saturated dicarboxylic acid having 16-28 carbons, and more than half of the hydroxyl groups of the condensate glycerin remaining as hydroxyl groups in the ester compound;
■ a component B: a dihydroxy water soluble alcohol; and
■ a component C: a polyhydroxy water soluble alcohol which is at least trihydroxy.

2. The humectant according to claim 1, wherein the ester compound as the component A is an ester compound comprising a condensate of glycerin having polymerization degree of 5 or more, a straight chain saturated fatty acid having 16-28 carbons, and an aliphatic saturated dicarboxylic acid having 16-28 carbons, and is obtained by an esterification reaction of them in a charge amount which enables more than half of the hydroxyl groups of the condensate of glycerin to remain as hydroxyl groups in the ester compound in case of ester bonding of the condensate of glycerin with the straight chain saturated fatty acid and the aliphatic saturated dicarboxylic acid.

3. The humectant according to claim 1 or 2, further comprising water.

4. The humectant according to claim 1 or 2, further comprising an oily substance.

5. The humectant according to claim 1or 2, further comprising water and an oily substance.

6. A gel humectant comprising the humectant of claim 1 or 2 and an oily substance.

7. A gel humectant comprising the humectant of claim 1 or 2, water and an oily substance.

8. An oil-in-water type emulsion humectant comprising the humectant of claim 1 or 2, water and an oily substance.

9. An oil-in-water type emulsion humectant obtained by adding water to the gel humectant of claim 6.

10. An oil-in-water type emulsion humectant obtained by adding water to the gel humectant of claim 7.

11. A solubilized humectant comprising the humectant of claim 1 or 2, water and an oily substance.

12. A solubilized humectant obtained by adding water to the gel humectant of claim 6.

13. A solubilized humectant obtained by adding water to the gel humectant of claim 7.

14. The humectant according to claim 1 or 2, wherein the dihydroxy water soluble alcohol as the component B is 1,3-butylene glycol.

15. The humectant according to claim 1 or 2, wherein the content of the component A is from 0.1 to 50% by mass based on 100% by mass of the resulting humectant and a mass ratio of the component B to the component C is from 1:0.1 to 1:20.

16. A cosmetic comprising the humectant of claim 1 or 2.

17. An external agent comprising the humectant of claim 1 or 2.

## Patentansprüche

1. Befeuchtungsmittel, das Folgendes umfasst:
■ einen Bestandteil A: eine Esterverbindung mit einem Glycerinkondensat mit einem Polymerisierungsgrad von 5 oder darüber, einer geradkettigen gesättigten Fettsäure mit 16 - 28 Kohlenstoffen, und einer aliphatischen gesättigten Dicarbonsäure mit 16-28 Kohlenstoffen, und wobei mehr als die Hälfte der Hydroxylgruppen des Glycerinkondensats als Hydroxylgruppen in der Esterverbindung verbleiben;
■ einen Bestandteil B: einen wasserlöslichen Dihydroxy-Alkohol; und
■ einen Bestandteil C: einen wasserlöslichen Polyhydroxy-Alkohol, der mindestens ein Trihydroxy-Alkohol ist.

2. Befeuchtungsmittel nach Anspruch 1, wobei die Esterverbindung als Bestandteil A eine Esterverbindung ist, die ein Glycerinkondensat mit einem Polymerisierungsgrad von 5 oder darüber, eine geradkettige gesättigte Fettsäure mit 16 - 28 Kohlenstoffen, und eine aliphatische gesättigte Dicarbonsäure mit 16 - 28 Kohlenstoffen umfasst, und durch eine Veresterungsreaktion von diesen in einer Füllmenge gewonnen wird, die es ermöglicht, dass mehr als die Hälfte der Hydroxylgruppen des Glycerinkondensats im Falle einer Esterbindung des Glycerinkondensats mit der geradkettigen gesättigten Fettsäure und der aliphatischen gesättigten Dicarbonsäure als Hydroxylgruppen in der Esterverbindung verbleiben.

3. Befeuchtungsmittel nach Anspruch 1 oder 2, darüber hinaus Wasser umfassend.

4. Befeuchtungsmittel nach Anspruch 1 oder 2, darüber hinaus einen ölhaltigen Stoff umfassend.

5. Befeuchtungsmittel nach Anspruch 1 oder 2, darüber hinaus Wasser und einen ölhaltigen Stoff umfassend.

6. Gel-Befeuchtungsmittel, welches das Befeuchtungsmittel nach Anspruch 1 oder 2 und einen ölhaltigen Stoff umfasst.

7. Gel-Befeuchtungsmittel, welches das Befeuchtungsmittel nach Anspruch 1 oder 2, Wasser und einen ölhaltigen Stoff umfasst.

8. Öl-in-Wasser-Emulsion-Befeuchtungsmittel, welches das Befeuchtungsmittel nach Anspruch 1 oder 2, Wasser und einen ölhaltigen Stoff umfasst.

9. Öl-in-Wasser-Emulsion-Befeuchtungsmittel, welches durch Zusetzen von Wasser zum Gel-Befeuchtungsmittel nach Anspruch 6 gewonnen wird.

10. Öl-in-Wasser-Emulsion-Befeuchtungsmittel, welches durch Zusetzen von Wasser zum Gel-Befeuchtungsmittel nach Anspruch 7 gewonnen wird.

11. Solubilisiertes Befeuchtungsmittel, welches das Befeuchtungsmittel nach Anspruch 1 oder 2, Wasser und einen ölhaltigen Stoff umfasst.

12. Solubilisiertes Befeuchtungsmittel, welches durch Zusetzen von Wasser zum Gel-Befeuchtungsmittel nach Anspruch 6 gewonnen wird.

13. Solubilisiertes Befeuchtungsmittel, welches durch Zusetzen von Wasser zum Gel-Befeuchtungsmittel nach Anspruch 7 gewonnen wird.

14. Befeuchtungsmittel nach Anspruch 1 oder 2, wobei der wasserlösliche Dihydroxy-Alkohol als Bestandteil B 1,3-Butylenglycol ist.

15. Befeuchtungsmittel nach Anspruch 1 oder 2, wobei der Gehalt des Bestandteils A 0,1 bis 50 Massen-% basierend auf 100 Massen-% des sich ergebenden Befeuchtungsmittels beträgt, und ein Massenverhältnis des Bestandteils B zum Bestandteil C 1:0,1 bis 1:20 beträgt.

16. Kosmetikum, welches das Befeuchtungsmittel nach Anspruch 1 oder 2 umfasst.

17. Äußerlich anzuwendendes Mittel, welches das Befeuchtungsmittel nach Anspruch 1 oder 2 umfasst.

## Revendications

1. Humectant comportant:
• un composant A : un composé ester comportant un condensat de glycérine ayant un degré de polymérisation de 5 ou plus, un acide gras saturé à chaîne rectiligne ayant 16 à 28 carbones, et un acide dicarboxylique saturé aliphatique ayant 16 à 28 carbones, et plus de la moitié des groupes hydroxyle de la glycérine de condensat demeurant sous forme de groupes hydroxyle dans le composé ester,
• un composant B : un alcool soluble dans l'eau à base de dihydroxy, et
• un composant C : un alcool soluble dans l'eau à base de polyhydroxy qui est au moins trihydroxy.

2. Humectant selon la revendication 1, dans lequel le composé ester en tant que composant A est un composé ester comportant un condensat d'une glycérine ayant un degré de polymérisation de 5 ou plus, un acide gras saturé à chaîne rectiligne ayant 16 à 28 carbones, et un acide dicarboxylique saturé aliphatique ayant 16 à 28 carbones, et est obtenu par une réaction d'estérification de ceux-ci selon une quantité de charge qui permet à plus de la moitié des groupes hydroxyle du condensat de glycérine de demeurer sous forme de groupes hydroxyle dans le composé ester dans le cas d'une liaison ester du condensat de glycérine avec l'acide gras saturé à chaîne rectiligne et l'acide dicarboxylique saturé aliphatique.

3. Humectant selon la revendication 1 ou 2, comportant de plus de l'eau.

4. Humectant selon la revendication 1 ou 2, comportant de plus une substance huileuse.

5. Humectant selon la revendication 1 ou 2, comportant de plus de l'eau et une substance huileuse.

6. Humectant gélifié comportant l'humectant selon la revendication 1 ou 2 et une substance huileuse

7. Humectant gélifié comportant l'humectant selon la revendication 1 ou 2, de l'eau et une substance huileuse.

8. Humectant en émulsion de type huile dans eau comportant l'humectant selon la revendication 1 ou 2, de l'eau et une substance huileuse.

9. Humectant en émulsion de type huile dans eau obtenu en ajoutant de l'eau à l'humectant gélifié selon la revendication 6.

10. Humectant en émulsion de type huile dans eau obtenu en ajoutant de l'eau à l'humectant gélifié selon la revendication 7.

11. Humectant solubilisé comportant l'humectant selon la revendication 1 ou 2, de l'eau et une substance huileuse.

12. Humectant solubilisé obtenu en ajoutant de l'eau à l'humectant gélifié selon la revendication 6.

13. Humectant solubilisé obtenu en ajoutant de l'eau à l'humectants gélifié selon la revendication 7.

14. Humectant selon la revendication 1 ou 2, dans lequel l'alcool soluble dans l'eau à base de dihydroxy en tant que composant B est 1,3-butylèneglycol.

15. Humectant selon la revendication 1 ou 2, dans lequel la teneur en composant A est de 0,1 à 50 % en masse sur la base de 100 % en masse de l'humectant résultant et un rapport en masse entre le composant B et le composant C est compris entre 1:0:1 1 et 1:20.

16. Produit cosmétique comportant l'humectant selon la revendication 1 ou 2.

17. Agent externe comportant l'humectant selon la revendication 1 ou 2.
